# FASCICULE DE BREVET EUROPEEN

(11) **EP 1 227 791 B1**
(45) Date de publication et mention de la délivrance du brevet: **08.11.2006**
(21) Numéro de dépôt: 00993481.1
(22) Date de dépôt: 21.12.2000
(51) Int. Cl.: A61K 8/368, A61K 31/192, A61Q 19/00, A61Q 19/08

(54) **UTILISATION DE COMPOSES POLYCYCLIQUES AROMATIQUES EN TANT QU'ACTIVATEURS DES RECEPTEURS DE TYPE PPARS DANS UNE COMPOSITION COSMETIQUE OU PHARMACEUTIQUE**
VERWENDUNG VON AROMATISCHEN POLYZYKLISCHEN VERBINDUNGEN ALS AKTIVATOREN FÜR DIE PPARS REZEPTOREN IN EINER KOSMETISCHEN ODER EINER PHARMAZEUTISCHEN ZUSAMMENSETZUNG
USE OF AROMATIC POLYCYCLIC COMPOUNDS AS ACTIVATORS OF PPARS-TYPE RECEPTORS IN A COSMETIC OR PHARMACEUTICAL COMPOSITION

(30) Priorité: 22.12.1999 FR 9916269
(43) Date de publication de la demande: 07.08.2002
(73) Titulaire: L'ORÉAL, 75008 Paris (FR)
(72) Inventeur: MAIGNAN, Jean, 93290 Tremblay-en-France (FR); MICHEL, Serge, 06330 Roquefort-les-Pins (FR)
(74) Mandataire: Renard, Emmanuelle
(86) Numéro de dépôt international: PCT/FR2000/003645
(87) Numéro de publication internationale: WO 2001/045663

(56) Documents cités:
- WO-A-93/03713
- US-A- 4 826 969
- US-A- 4 829 080
- US-A- 4 892 940
- US-A- 5 004 732
- US-A- 5 023 363
- US-A- 6 001 885
- CHEN S ET AL: "RETINOIC ACID RECEPTOR GAMMA MEDIATES TOPICAL RETINOID EFFICACY AND IRRITATION IN ANIMAL MODELS" JOURNAL OF INVESTIGATIVE DERMATOLOGY,US,NEW YORK, NY, vol. 104, no. 5, 1 mai 1995 (1995-05-01), pages 779-783, XP000600681 ISSN: 0022-202X

## Description

La présente invention concerne une utilisation de composés pour la fabrication d'une composition destinée à traiter des désordres cutanés choisis parmi la rosacée, le vieillissement intrinsèque (ou chronologique), des désordres de la pigmentation, , des désordres de la fonction barrière et plus particulièrement les désordres de la sécrétion des lipides épidermiques, des troubles de la cicatrisation, des atrophies cutanées induites par les corticostéroïdes ou des ulcères ; des troubles du système immunitaire ; des affections du système cardiovasculaire, et/ou des troubles du métabolisme des lipides.

La présente invention concerne également un procédé de traitement cosmétique pour restaurer la fonction barrière et plus particulièrement pour réguler et/ou restaurer le métabolisme des lipides cutanés caractérisé en ce qu'on applique sur la peau une composition comprenant au moins un composé de formule (I), et plus particulièrement en tant qu'activateur des récepteurs de type PPARs.

De manière tout à fait surprenante et inattendue, la Demanderesse a trouvé que certains composés décrits dans les demandes de brevet EP 220 118, US 5 023 363, FR 2 600 064 comme composés ayant une action antiproliférative ont une activité marquée vis à vis de la transactivation des récepteurs de type PPARs.

Cette découverte est à la base de la présente invention.

La présente invention a donc trait à une utilisation d'une quantité efficace d'au moins un composé, et plus particulièrement en tant qu'activateurs des récepteurs de type PPARs, pour la fabrication d'une composition destinée à traiter des désordres cutanés choisis parmi la rosacée, le vieillissement intrinsèque (ou chronologique), des désordres de la pigmentation, des désordres de la fonction barrière et plus particulièrement les désordres de la sécrétion des lipides épidermiques, des troubles de la cicatrisation, des atrophies cutanées induites par les corticostéroïdes ou des ulcères ; des troubles du système immunitaire ; des affections du système cardiovasculaire, et/ou des troubles du métabolisme des lipides.

La présente invention concerne également un procédé de traitement cosmétique pour restaurer la fonction barrière et plus particulièrement pour réguler et/ou restaurer le métabolisme des lipides cutanés caractérisé en ce qu'on applique sur la peau une composition comprenant au moins un composé de formule (I), et plus particulièrement en tant qu'activateur des récepteurs de type PPARs.

Ces composés présentent une formule générale (I): 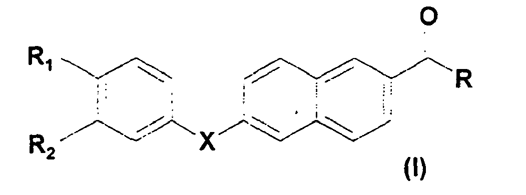 dans laquelle
R représente un atome d'hydrogène ou un radical -OR₃
R₃ ayant les significations données ci-après,
X représente un radical C=O, ou un radical C=N-OH,
R₁ et R₂ identiques ou différents représentent un atome d'hydrogène, un radical alkyle linéaire ou ramifié ayant de 1 à 12 atomes de carbone, un radical -OR₄, un radical -S(O)ₙR₄, un radical -OCOR₄, ou un radical -NHCOR₄,
n, R₄ ayant les significations données ci-après,
R₁ et R₂ pris ensemble peuvent former avec le cycle aromatique adjacent un cycle saturé ou insaturé à 5 ou 6 chaînons éventuellement substitué par des groupes méthyle et/ou éventuellement interrompu par un atome d'oxygène, de soufre, un radical sulfone, ou un radical sulfoxyde, de préférence le cycle est saturé,
étant entendu que lorsque X représente un radical C=O, alors R1 et R2 ne forment pas avec le cycle aromatique adjacent un cycle à 5 chaînons substitué par des groupes méthyles,
R₃ représente un atome d'hydrogène, un radical alkyle linéaire ou ramifié ayant de 1 à 20 atomes de carbone, ou un radical mono ou polyhydroxyalkyle, n représente 0, 1 ou 2,
R₄ représente un atome d'hydrogène, un radical alkyle inférieur, ou un radical phényle,
ainsi que leurs sels, et leurs analogues chiraux. Les composés de formule (I) peuvent se présenter sous forme de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Parmi les radicaux alkyle linéaire ou ramifié ayant de 1 à 12 atomes de carbone, on peut notamment citer les radicaux méthyle, éthyle, propyle, 2-éthyl-hexyle, octyle, dodécyle.

Parmi les radicaux alkyle linéaire ou ramifié ayant de 1 à 20 atomes de carbone, on peut notamment citer les radicaux méthyle, éthyle, propyle, 2-éthyl-hexyle, octyle, dodécyle, hexadécyle et octadécyle.

Par radical monohydroxyalkyle, on entend un radical ayant 1 à 6 atomes de carbone et de préférence ayant de 2 à 3 atomes de carbone, notamment un radical 2-hydroxyéthyle, 2-hydroxypropyle ou 3-hydroxypropyle.

Par radical polyhydroxyalkyle, on entend un radical contenant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les composés de formule (I) ci-dessus, on peut notamment citer les suivants:
Composé 1: acide 6-(5,6,7,8-Tetrahydro-5,5,8,8-tetramethylnaphthalene-2-carbonyl)-naphthalene-2-carboxylique
Composé 2: acide 6-(2,2-Dimethyl-chroman-6-carbonyl)-naphthalene-2-carboxylique
Composé 3 : acide 6-(4-tert-Butyl-benzoyl)-naphthalene-2-carboxylique
Composé 4 : acide 6-(5,6,7,8-Tetrahydro-naphthalene-2-carbonyl)-naphthalene-2-carboxylique
Composé 5 : acide 6-(4,4-Dimethyl-chroman-6-carbonyl)-naphthalene-2-carboxylique
Composé 6: acide 6-[Hydroxyimino-(5,5,8,8-tetramethyl-5,6,7,8-tetrahydro-naphthalen-2-yl)-methyl]-naphthalene-2-carboxylique

Selon la présente invention le composé de formule (I) plus particulièrement utilisé est le composé 3 : l'acide 6-(4-tert-Butyl-benzoyl)-naphthalene-2-carboxylique.

Les composés de formule (I) peuvent être notamment obtenus par les procédés de préparation décrits dans les demandes de brevets EP 220 118, US 5 023 363 et FR 2 600 064.

Les composés de l'invention présentent des propriétés d'activation des récepteurs de type PPARs. Les récepteurs de type PPARs sont des récepteurs qui appartiennent à la famille des récepteurs nucléaires stéroïdiens.

Par activateur des récepteurs de type PPARs, on entend selon l'invention tout composé qui présente dans un test de transactivation, tel que décrit dans Kliewer et al., Nature 358, 771-774, 1992, une AC50 inférieure ou égale à 10 µM. De préférence, l'activateur des récepteurs de type PPAR présente une AC50 inférieure ou égale à 2 µM et avantageusement inférieure ou égale à 1 µM.

Une AC50 est la concentration en composé "activateur" nécessaire pour présenter 50% de l'activité d'une molécule de référence. Cette activité est déterminée à l'aide d'une enzyme (luciférase) rapporteuse de l'activation due au composé via un des récepteurs PPARs.

L'activité des récepteurs de type PPARs a fait l'objet de nombreuses études. On peut citer à titre indicatif la publication intitulée "Differential Expression of Peroxisome Proliferator-Activated Receptor Subtypes During the Differentiation of Human Keratinocytes", Michel Rivier et al., J. Invest. Dermatol 111, 1998, p 1116-1121, dans laquelle sont répertoriées un grand nombre de références bibliographiques concernant les récepteurs de type PPARs.

L'utilisation des activateurs des récepteurs de type PPAR-α pour restaurer la fonction barrière et plus particulièrement les désordres de la sécrétion des lipides épidermiques, promouvoir la différentiation et inhiber la prolifération épidermique a été décrite dans la demande de brevet internationale WO 98/32444.
De plus, l'utilisation des activateurs des récepteurs de type PPAR-α et/ou PPAR-γ pour traiter les désordres cutanés liés à une anomalie de la différenciation des cellules épidermiques a été décrite dans la publication de Michel Rivier et al., J. Invest. Dermatol 111, 1998, p 1116-1121.

Il a également été décrit dans la demande de brevet WO 96/33724 que des composés sélectifs des PPARγ, tels qu'une prostaglandine-J2 ou -D2 sont des actifs potentiels pour le traitement de l'obésité et du diabète.

Les compositions pharmaceutiques contenant au moins un composé de formule (I) sont donc destinées au traitement de la rosacée, des désordres de la pigmentation, des désordres de la fonction barrière et plus particulièrement les désordres de la sécrétion des lipides épidermiques, des troubles de la cicatrisation, des atrophies cutanées induites par les corticostéroïdes ou des ulcères ; des troubles du système immunitaire ; des affections du système cardiovasculaire, et/ou des troubles du métabolisme des lipides.

Par désordres de la pigmentation, on entend notamment l'hyperpigmentation, le mélasma, l'hypopygmentation ou le vilitigo. De préférence, les désordres de la pigmentation ne sont pas liés à des effets néfastes du soleil.

Parmi les désordres de la fonction barrière et plus particulièrement les désordres de la sécrétion des lipides épidermiques, on peut citer notamment les désordres de la peau des enfants prématurés nés avant 33 semaines, les fissures labiales ou les bulles suite à friction mécanique.

Parmi les troubles de la cicatrisation, on peut citer notamment les kéloïdes ou les cicatrices hypertrophiques.

Parmi les ulcères, on peut citer notamment les ulcères et érosions dus à des brûlures chimiques ou thermiques, les désordres bulleux, ou les troubles vasculaires ou ischémie incluant les ulcères veineux, artériels, emboliques ou diabétiques.

Parmi les troubles du système immunitaire , on peut citer notamment les maladies auto-immunes (comme, mais sans limitation, le diabète sucré de type 1, la sclérose en plaques, le lupus et les affections de type lupus, la glomérulonéphrite, etc.), ou des dysfonctionnements sélectifs du système immunitaire (par exemple le SIDA).

Parmi les affections du système cardiovasculaire, on peut citer notamment l'athérosclérose ou l'hypertension.

Parmi les affections du métabolisme des lipides, on peut citer l'obésité, l'hyperlipidémie, ou le diabète non insulino-dépendant.

L'administration de la composition selon l'invention peut être effectuée par voie entérale, parentérale topique ou oculaire. De préférence, la composition pharmaceutique est conditionnée sous une forme convenant à une application par voie topique.

Par voie entérale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Les composés sont utilisés selon l'invention sont généralement administrés à une dose journalière d'environ 0,001 mg/kg à 100 mg/kg en poids corporel en 1 à 3 prises.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter soit sous forme anhydre, soit sous forme aqueuse.

Les composés sont utilisés par voie topique à une concentration généralement comprise entre 0,001 % et 10 % en poids, de préférence entre 0,01 et 1 % en poids, par rapport au poids total de la composition.

Les composés de formule (I) selon l'invention, trouvent également une application dans le domaine cosmétique, en particulier dans l'hygiène corporelle et capillaire et plus particulièrement pour réguler et/ou restaurer le métabolisme des lipides cutanés. Par rapport aux produits connus antérieurement, ces composés de formule (I) ont l'avantage de présenter en plus d'autres propriétés intéressantes, notamment des propriétés anti-inflammatoires ou apaisantes, ce qui en fait des composés moins irritants et donc mieux tolérés.

La composition cosmétique selon l'invention contenant, dans un support cosmétiquement acceptable, au moins un composé de formule (I) l'un de ses isomères optiques ou géométriques ou l'un de ses sels, peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques est comprise entre 0,001 et 3 % en poids.

D'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

Les compositions telles que décrites précédemment peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; les acides eicosa-5,8,11,14-tétraynoïque et eicosa-5,8,11-triynoïque, leurs esters et amides et enfin les rétinoïdes. Les composés de formule (I) peuvent également être combinés avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.
Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre d'illustration et sans aucun caractère limitatif, plusieurs exemples de résultats de tests biologiques de composés actifs de formule (I) selon l'invention, ainsi que diverses formulations concrètes à base de tels composés. Dans ce qui suit ou ce qui précède, les pourcentages sont donnés en poids sauf mention contraire.

### EXEMPLE 1

Dans cet exemple, on a illustré divers résultats de tests biologiques qui montrent les propriétés de transactivation des récepteurs PPARs des composés de l'invention.

Les exemples comparatifs correspondent à des composés qui sont décrits dans les demandes de brevet EP 220 118 (exemples comparatifs 1 et 2), ou US 5 023 363 (exemples comparatifs 3 à 7), mais qui ne vérifient pas les conditions des composés de formule (I).
Les tests biologiques effectués correspondent à ceux décrits dans la demande. La méthode utilisée pour déterminer les AC50 est celle décrite dans Kliewer et al., Nature 358, 771-774, 1992. Ainsi, le pouvoir activateur via PPAR-α, PPAR-γ ou PPAR-δ, de molécules peut être évalué avec un test de transactivation dans lequel les cellules HeLa ont été cotransfectées par un vecteur d'expression codant pour ces récepteurs et un plasmide rapporteur contenant un élément de réponse PPRE cloné en amont d'une partie d'un promoteur du virus SV40 et du gène luciférase. Les cellules cotransfectées sont traitées pendant 24 heures avec les molécules à tester et l'activité de la luciférase est déterminée par luminescence.
La référence 1, molécule de référence des PPAR-α est l'acide [4-Chloro-6(2,3-dimethyl-phenylamino)-pyrimidin-2-ylsulfanyl]acetique;
La référence 2, molécule de référence des PPAR-δ et PPAR-γ est la 5-{4[2-(methyl-pyridin-2-yl-amino)-éthoxy]-benzyl}-thiazolidine-2,4-dione;

L'exemple comparatif 1 est l'acide 6-[Butoxy-(5,5,8,8-tetramethyl-5,6,7,8-tetrahydro-naphthalen-2-yl)-methyl]-naphthalene-2-carboxylique;
L'exemple comparatif 2 est le C-(6-Carboxy-naphthalen-2-yl)-C-(5,5,8,8-tetramethyl-5,6,7,8-tetrahydro-naphthalen-2-yl)- methyl-ammonium ;
L'exemple comparatif 3 est l'acide 6-(2,4-Diisopropyl-benzoyl)-naphthalene-2-carboxylique ;
L'exemple comparatif 4 est l'acide 6-(6-Methoxy-biphenyl-3-carbonyl)-naphthalene-2-carboxylique ;
L'exemple comparatif 5 est l'acide 6-(4-Adamantan-1-yl-3-methoxy-benzoyl)-naphthalene-2-carboxylique ;
L'exemple comparatif 6 est l'acide 4-[(3-Adamantan-1-yl-4-methoxy-phenyl)-hydroxy-methyl]-benzoique ;

L'exemple comparatif 7 est l'acide 4-(3-Adamantan-1-yl-4-methoxy-benzoyl)-benzoique.

Les résultats obtenus dans les tests de transactivation des récepteurs de type PPARs sont regroupés dans le tableau suivant :

| composés | α | γ | β |
|---|---|---|---|
| Référence 1 | 100*(1,4)** | n.a | n.a |
| Référence 2 | n.a | 100(0,07) | 100(0,13) |
| Composé 1 | 10 | 10 | 64 |
| Composé 2 | 48 (0,9) | 190 (1,5) | 280 (0,7) |
| Composé 3 | 63 (1) | 148 (0,36) | 277 (0,3) |
| Composé 4 | 40 (2) | 174 (0,8) | 291 (0,5) |
| Composé 5 | 16 | 36 | 92 |
| Composé 6 | 7 | 20 | 54 |
| Ex comparatif 1 | 0 | 7 | 28 |
| Ex comparatif 2 | 1 | 13 | 1 |
| Ex comparatif 3 | 6 | 3 | 19 |
| Ex comparatif 4 | 6 | 2 | 23 |
| Ex comparatif 5 | 6 | 3 | 8 |
| Ex comparatif 6 | 4 | 7 | 16 |
| Ex comparatif 7 | 0 | 0 | 23 |

| | | | |
|---|---|---|---|
| n.a signifie non actif * % d'activation ()** AC₅₀ en µM | | | |

Ces résultats montrent l'activation des composés de l'invention pour les différents sous-types de récepteurs de type PPARs : PPAR-α, PPAR-β, et PPAR-γ.

### EXEMPLE 2

Dans cet exemple, on a illustré diverses formulations concrètes à base des composés selon l'invention.

### A- VOIE ORALE

(a) Comprimé de 0,2 g
   - Composé 1 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé 5 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé 2 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé 4 0,200 g
   - Glycérine 1,000 g
   - Sorbitol à 70% 1,000 g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé 6 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé 2 0,300 g
   - Vaseline blanche codex qsp 100 g
(c) Crème Eau-dans-Huile non ionique
   - Composé 1 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé 3 0,100 g
   - Polyéthylène glycol (PEG 400) 69,900 g
   - Ethanol à 95% 30,000 g
(e) Onguent hydrophobe
   - Composé 5 0,300 g
   - Miristate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 cst" vendu par GOLDSCHMIDT) qsp 100 g
(f) Crème Huile-dans-Eau non ionique
   - Composé 2 1,000 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Utilisation d'une quantité efficace d'au moins un composé de formule générale (I) : 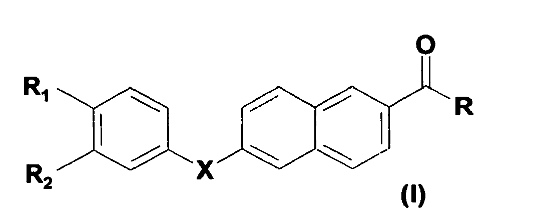 dans laquelle
R représente un atome d'hydrogène ou un radical -OR₃
R₃ ayant les significations données ci-après,
X représente un radical C=O, ou un radical C=N-OH,
R₁ et R₂ identiques ou différents représentent un atome d'hydrogène, un radical alkyle linéaire ou ramifié ayant de 1 à 12 atomes de carbone, un radical -OR₄, un radical -S(O)ₙR₄, un radical -OCOR₄, ou un radical -NHCOR₄,
n, R₄ ayant les significations données ci-après,
R₁ et R₂ pris ensemble peuvent former avec le cycle aromatique adjacent un cycle à 5 ou 6 chaînons éventuellement substitué par des groupes méthyle et/ou éventuellement interrompu par un atome d'oxygène, de soufre, un radical sulfone, ou un radical sulfoxyde,
étant entendu que lorsque X représente un radical C=O, alors R1 et R2 ne forment pas avec le cycle aromatique adjacent un cycle à 5 chaînons substitué par des groupes méthyles,
R₃ représente un atome d'hydrogène, un radical alkyle linéaire ou ramifié ayant de 1 à 20 atomes de carbone, ou un radical mono ou polyhydroxyalkyle,
n représente 0, 1 ou 2,
R₄ représente un atome d'hydrogène, un radical alkyle inférieur, ou un radical phényle,
ainsi que leurs sels, et leurs analogues chiraux
pour la fabrication d'une composition pharmaceutque destinée à traiter des désordres cutanés choisis parmi la rosacée, des désordres de la pigmentation, des désordres de la fonction barrière et plus particulièrement les désordres de la sécrétion des lipides épidermiques, des troubles de la cicatrisation, des atrophies cutanées induites par les corticostéroïdes ou des ulcères ; des troubles du système immunitaire ; des affections du système cardiovasculaire, et/ou des troubles du métabolisme des lipides.

2. Utilisation cosmétique d'une quantité efficace d'au moins un composé de formule (I) : 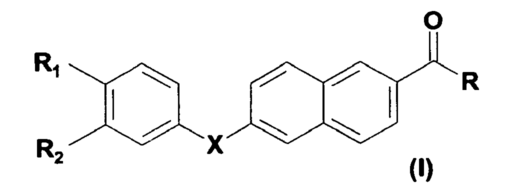 dans laquelle
R représente un atome d'hydrogène ou un radical -OR₃
R₃ ayant les significations données ci-après,
X représente un radical C=O, ou un radical C=N-OH,
R₁ et R₂ identiques ou différents représentent un atome d'hydrogène, un radical alkyle linéaire ou ramifié ayant de 1 à 12 atomes de carbone, un radical -OR₄, un radical -S(O)ₙR₄, un radical -OCOR₄, ou un radical -NHCOR₄,
n, R₄ ayant les significations données ci-après,
R₁ et R₂ pris ensemble peuvent former avec le cycle aromatique adjacent un cycle à 5 ou 6 chaînons éventuellement substitué par des groupes méthyle et/ou éventuellement interrompu par un atome d'oxygène, de soufre, un radical sulfone, ou un radical sulfoxyde,
étant entendu que lorsque X représente un radical C=O, alors R1 et R2 ne forment pas avec le cycle aromatique adjacent un cycle à 5 chaînons substitué par des groupes méthyles,
R₃ représente un atome d'hydrogène, un radical alkyle linéaire ou ramifié ayant de 1 à 20 atomes de carbone, ou un radical mono ou polyhydroxyalkyle,
n représente 0, 1 ou 2,
R₄ représente un atome d'hydrogène, un radical alkyle inférieur, ou un radical phényle,
ainsi que leurs sels, et leurs analogues chiraux
pour prévenir et/ou traiter le vieillissement intrinsèque (ou chronologique).

3. Utilisation selon la revendication 1 ou 2, **caractérisée en ce que** les composés de formule (I) sont utilisés en tant qu'activateurs des récepteurs de type PPARs

4. Utilisation selon l'une quelconque des revendications 1 à 3, **caractérisée en ce que** les composés de formule (I) se présentent sous forme de sels d'un métal alcalin ou alcalino-terreux, ou encore de zinc ou d'une amine organique.

5. Utilisation selon l'une quelconque des revendications 1 à 4, **caractérisée en ce que** les radicaux alkyles inférieurs sont choisis dans le groupe constitué par les radicaux méthyle, éthyle, isopropyle, butyle, tertiobutyle et hexyle.

6. Utilisation selon l'une quelconque des revendications précédentes, **caractérisée en ce que** les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyle.

7. Utilisation selon l'une quelconque des revendications précédentes, **caractérisée en ce que** les radicaux monohydroxyalkyles sont choisis dans le groupe constitué par les radicaux 2-hydroxyéthyle, 2-hydroxypropyle ou 3-hydroxypropyle.

8. Utilisation selon l'une quelconque des revendications précédentes, **caractérisée en ce que** les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

9. Utilisation selon l'une quelconque des revendications 1 ou 2, **caractérisée par le fait que** les composés sont choisis dans le groupe constitué par :
Composé 1 : acide 6-(5,6,7,8-Tetrahydro-5,5,8,8-tetramethylnaphthalene-2-carbonyl)-naphthalene-2-carboxylique
Composé 2: acide 6-(2,2-Dimethyl-chroman-6-carbonyl)-naphthalene-2-carboxylique
Composé 3 : acide 6-(4-tert-Butyl-benzoyl)-naphthalene-2-carboxylique
Composé 4: acide 6-(5,6,7,8-Tetrahydro-naphthalene-2-carbonyl)-naphthalene-2-carboxylique
Composé 5: acide 6-(4,4-Dimethyl-chroman-6-carbonyl)-naphthalene-2-carboxylique
Composé 6: acide 6-[Hydroxyimino-(5,5,8,8-tetramethyl-5,6,7,8-tetrahydro-naphthalen-2-yl)-methyl]-naphthalene-2-carboxylique

10. Utilisation selon l'une quelconque des revendications 1 ou 2, **caractérisée en ce que** le composé correspondant à la formule (I) est l'acide 6-(4-tert-Butyl-benzoyl)-naphthalene-2-carboxylique.

11. Utilisation selon l'une quelconque des revendications 1 ou 3, **caractérisée en ce que** les désordres de la pigmentation sont l'hyperpigmentation, le mélasma, l'hypopygmentation ou le vilitigo.

12. Utilisation selon la revendication 11, **caractérisée en ce que** les désordres de la pigmentation ne sont pas liés à des effets néfastes du soleil.

13. Utilisation selon l'une quelconque des revendications 1 ou 3, **caractérisée en ce que** les troubles de la fonction séborrhée sont l'hyperséborrhée ou la dermite séborrhéique.

14. Utilisation selon l'une quelconque des revendications 1 ou 3, **caractérisée en ce que** les désordres de la fonction barrière et plus particulièrement les désordres de la sécrétion des lipides épidermiques sont les désordres de la peau des enfants prématurés nés avant 33 semaines, les fissures labiales ou les bulles suite à friction mécanique.

15. Utilisation selon l'une quelconque des revendications 1 ou 3, **caractérisée en ce que** les troubles de la cicatrisation sont les kéloïdes ou les cicatrices hypertrophiques.

16. Utilisation selon l'une quelconque des revendications 1 ou 3, **caractérisée en ce que** les ulcères sont les ulcères et érosions dus à des brûlures chimiques ou thermiques, les désordres bulleux, ou les troubles vasculaires ou ischémie incluant les ulcères veineux, artériels, emboliques ou diabétiques.

17. Utilisation selon l'une quelconque des revendications 1 ou 3, **caractérisée en ce que** les troubles du système immunitaire sont les maladies auto-immunes comme le diabète sucré de type 1, la sclérose en plaques, le lupus et les affections de type lupus, ou la glomérulonéphrite, ou des dysfonctionnements sélectifs du système immunitaire tel que le SIDA.

18. Utilisation selon l'une quelconque des revendications 1 ou 3, **caractérisée en ce que** les affections du système cardiovasculaire sont l'athérosclérose ou l'hypertension.

19. Utilisation selon l'une quelconque des revendications 1 ou 3, **caractérisée en ce que** les troubles du métabolisme des lipides sont l'obésité, l'hyperlipidémie, ou le diabète non insulino-dépendant.

20. Utilisation selon l'une quelconque des revendications précédentes, **caractérisée en ce que** les composés de formule (I) sont combinés avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques.

21. Utilisation selon l'une quelconque des revendications précédentes, **caractérisée en ce que** la composition est administrée par voie entérale ou parentérale.

22. Utilisation selon l'une quelconque des revendications 1 à 17, **caractérisée en ce que** la composition est administrée par voie topique ou oculaire.

23. Utilisation selon la revendication précédente, **caractérisée en ce que** les composés de formule (I) sont utilisés à une concentration comprise entre 0,001% et 10% en poids par rapport au poids total de la composition.

24. Procédé de traitement cosmétique pour restaurer la fonction barrière et plus particulièrement pour réguler et/ou pour restaurer le métabolisme des lipides cutanés **caractérisé en ce qu'**on applique sur la peau une composition comprenant au moins un composé de formule (I) dans une quantité efficace.

25. Procédé de traitement cosmétique pour restaurer la fonction barrière et plus particulièrement pour réguler et/ou pour restaurer le métabolisme des lipides cutanés **caractérisé en ce qu'**on applique sur la peau une composition comprenant au moins un composé de formule (I) en tant qu'activateur des récepteurs de type PPARs dans une quantité efficace.

26. Procédé de traitement cosmétique selon l'une quelconque des revendications 24 ou 25, **caractérisée en ce que** la concentration en composés de formule (I) est comprise entre 0,001% et 3% en poids par rapport à l'ensemble de la composition.

## Claims

1. Use of an effective quantity of at least one compound of general formula (I): 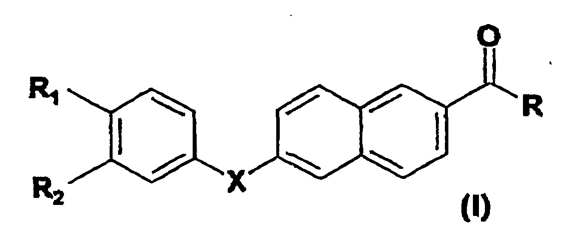 in which
R represents a hydrogen atom or an -OR₃ radical
R₃ having the meanings given below,
X represents a C=O radical or a C=N-OH radical,
R₁ and R₂, which are identical or different, represent a hydrogen atom, a linear or branched alkyl radical having from 1 to 12 carbon atoms, an -OR₄ radical, an -S(O)ₙR₄ radical, an -OCOR₄ radical or an -NHCOR₄ radical,
n and R₄ having the meanings given below,
R₁ and R₂, taken together, may form with the adjacent aromatic ring a 5- or 6-membered ring optionally substituted with methyl groups and/or optionally interrupted by an oxygen or sulphur atom, a sulphone radical or a sulphoxide radical,
it being understood that when X represents a C=O radical, then R₁ and R₂ do not form with the adjacent aromatic ring a 5-membered ring substituted with methyl groups,
R₃ represents a hydrogen atom, a linear or branched alkyl radical having from 1 to 20 carbon atoms or a mono- or polyhydroxyalkyl radical,
n represents 0, 1 or 2,
R₄ represents a hydrogen atom, a lower alkyl radical or a phenyl radical,
as well as their salts, and their chiral analogues
for the manufacture of a pharmaceutical composition intended for treating skin disorders chosen from rosacea, pigmentation disorders, barrier function disorders and more particularly epidermal lipid secretion disorders, wound healing disorders, corticosteroid-induced cutaneous atrophies or ulcers; immune system disorders; cardiovascular system conditions, and/or lipid metabolism disorders.

2. Cosmetic use of an effective quantity of at least one compound of formula (I): 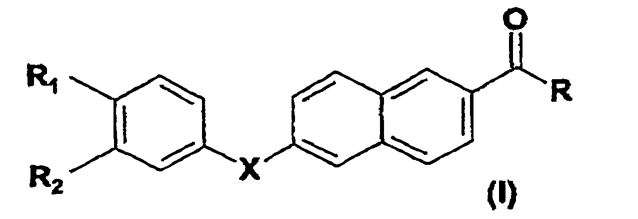 in which
R represents a hydrogen atom or an -OR₃ radical
R₃ having the meanings given below,
X represents a C=O radical or a C=N-OH radical,
R₁ and R₂, which are identical or different, represent a hydrogen atom, a linear or branched alkyl radical having from 1 to 12 carbon atoms, an -OR₄ radical, an -S(O)ₙR₄ radical, an -OCOR₄ radical or an -NHCOR₄ radical,
n and R₄ having the meanings given below,
R₁ and R₂, taken together, may form with the adjacent aromatic ring a 5- or 6-membered ring optionally substituted with methyl groups and/or optionally interrupted by an oxygen or sulphur atom, a sulphone radical or a sulphoxide radical,
it being understood that when X represents a C=O radical, then R₁ and R₂ do not form with the adjacent aromatic ring a 5-membered ring substituted with methyl groups,
R₃ represents a hydrogen atom, a linear or branched alkyl radical having from 1 to 20 carbon atoms or a mono- or polyhydroxyalkyl radical,
n represents 0, 1 or 2,
R₄ represents a hydrogen atom, a lower alkyl radical or a phenyl radical,
as well as their salts, and their chiral analogues
for preventing and/or treating intrinsic (or chronological) ageing.

3. Use according to Claim 1 or 2, **characterized in that** the compounds of formula (I) are used as PPAR-type receptor activators.

4. Use according to any one of Claims 1 to 3, **characterized in that** the compounds of formula (I) are provided in the form of salts of an alkali or alkaline-earth metal, or alternatively of zinc or of an organic amine.

5. Use according to any one of Claims 1 to 4, **characterized in that** the lower alkyl radicals are chosen from the group consisting of methyl, ethyl, isopropyl, butyl, tert-butyl and hexyl radicals.

6. Use according to any one of the preceding claims, **characterized in that** the linear or branched alkyl radicals having from 1 to 20 carbon atoms are chosen from the group consisting of methyl, ethyl, propyl, 2-ethylhexyl, octyl, dodecyl, hexadecyl and octadecyl radicals.

7. Use according to any one of the preceding claims, **characterized in that** the monohydroxyalkyl radicals are chosen from the group consisting of 2-hydroxyethyl, 2-hydroxypropyl or 3-hydroxypropyl radicals.

8. Use according to any one of the preceding claims, **characterized in that** the polyhydroxyalkyl radicals are chosen from the group consisting of 2,3-dihydroxypropyl, 2,3,4-trihydroxybutyl or 2,3,4,5-tetrahydroxypentyl radicals or the pentaerythritol residue.

9. Use according to either of Claims 1 and 2, **characterized in that** the compounds are chosen from the group consisting of:
Compound 1: 6-(5,6,7,8-tetrahydro-5,5,8,8-tetra-methylnaphthalene-2-carbonyl)naphthalene-2-carboxylic acid
Compound 2: 6-(2,2-dimethylchroman-6-carbonyl)-naphthalene-2-carboxylic acid
Compound 3: 6-(4-tert-butylbenzoyl)naphthalene-2-carboxylic acid
Compound 4: 6-(5,6,7,8-tetrahydronaphthalene-2-carbonyl)naphthalene-2-carboxylic acid
Compound 5: 6-(4,4-dimethylchroman-6-carbonyl)-naphthalene-2-carboxylic acid
Compound 6: 6-[hydroxyimino(5,5,8,8-tetramethyl-5,6,7,8-tetrahydronaphthalen-2-yl)methylnaphthalene-2-carboxylic acid

10. Use according to either of Claims 1 and 2, **characterized in that** the compound corresponding to formula (I) is 6-(4-tert-butylbenzoyl)-naphthalene-2-carboxylic acid.

11. Use according to either of Claims 1 and 3, **characterized in that** the pigmentation disorders are hyperpigmentation, melasma, hypopigmentation or vilitigo.

12. Use according to Claim 11, **characterized in that** the pigmentation disorders are not linked to harmful effects of the sun.

13. Use according to either of Claims 1 and 3, **characterized in that** the seborrhoeic function disorders are hyperseborrhoea or seborrhoeic dermatitis.

14. Use according to either of Claims 1 and 3, **characterized in that** the barrier function disorders and more particularly the epidermal lipid secretion disorders are skin disorders in premature children born before 33 weeks, lip fissures or blisters following mechanical friction.

15. Use according to either of Claims 1 and 3, **characterized in that** the wound healing disorders are keloids or hypertrophic scars.

16. Use according to either of Claims 1 and 3, **characterized in that** the ulcers are ulcers and erosions due to chemical or heat burns, bullous disorders, or vascular disorders or ischaemia including venous, arterial, embolic or diabetic ulcers.

17. Use according to either of Claims 1 and 3, **characterized in that** the immune system disorders are autoimmune diseases such as type 1 diabetes mellitus, multiple sclerosis, lupus and lupus-type conditions, or glomerulonephritis, or selective immune system dysfunctions such as AIDS.

18. Use according to either of Claims 1 and 3, **characterized in that** the cardiovascular system conditions are atherosclerosis or hypertension.

19. Use according to either of Claims 1 and 3, **characterized in that** the lipid metabolism disorders are obesity, hyperlipidaemia or non-insulin-dependent diabetes.

20. Use according to any one of the preceding claims, **characterized in that** the compounds of formula (I) are combined with other compounds with retinoid-type activity, with D vitamins or their derivatives, with corticosteroids, with anti-free radicals, α-hydroxy or α-keto acids or their derivatives, or alternatively with ion channel blockers.

21. Use according to any one of the preceding claims, **characterized in that** the composition is administered enterally or parenterally.

22. Use according to any one of Claims 1 to 17, **characterized in that** the composition is administered topically or ocularly.

23. Use according to the preceding claim, **characterized in that** the compounds of formula (I) are used at a concentration of between 0.001% and 10% by weight relative to the total weight of the composition.

24. Method of cosmetic treatment for restoring the barrier function and more particularly for regulating and/or for restoring the metabolism of cutaneous lipids, **characterized in that** a composition comprising at least one compound of formula (I) is applied to the skin in an effective quantity.

25. Method of cosmetic treatment for restoring the barrier function and more particularly for regulating and/or for restoring the metabolism of cutaneous lipids, **characterized in that** a composition comprising at least one compound of formula (I) is applied to the skin as a PPAR-type receptor activator in an effective quantity.

26. Method of cosmetic treatment according to either of Claims 24 and 25, **characterized in that** the concentration of compounds of formula (I) is between 0.001% and 3% by weight relative to the whole composition.

## Patentansprüche

1. Verwendung einer wirksamen Menge mindestens einer Verbindung der allgemeinen Formel (I): 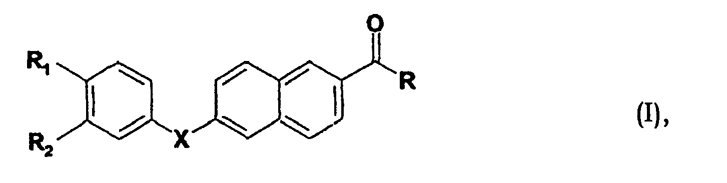 worin bedeuten:
R ein Wasserstoffatom oder eine Gruppe -OR₃,
wobei R₃ die unten angegebenen Bedeutungen hat,
X eine Gruppe C=O oder eine Gruppe C=N-OH,
R₁ und R₂, die gleich oder verschieden sind, ein Wasserstoffatom, eine geradkettige oder verzweigte Alkylgruppe mit 1 bis 12 Kohlenstoffatomen, eine Gruppe -OR₄, eine Gruppe -S(O)ₙR₄, eine Gruppe -OCOR₄ oder eine Gruppe NHCOR₄,
wobei n und R₄ die unten angegebenen Bedeutungen haben,
wobei R₁ und R₂ zusammen mit dem benachbarten aromatischen Ring einen 5- oder 6-gliedrigen Ring bilden können, der gegebenenfalls mit Methylgruppen substituiert ist und/oder gegebenenfalls durch ein Sauerstoffatom, ein Schwefelatom, eine Sulfongruppe oder eine Sulfoxidgruppe unterbrochen ist,
mit der Maßgabe, dass R 1 und R2 mit dem benachbarten aromatischen Ring keinen 5-gliedrigen Ring bilden, der mit Methylgruppen substituiert ist, wenn X eine C=O-Gruppe bedeutet,
R₃ ein Wasserstoffatom, eine geradkettige oder verzweigte Alkylgruppe mit 1 bis 20 Kohlenstoffatomen oder eine Mono- oder Polyhydroxyalkylgruppe,
n 0, 1 oder 2,
R₄ ein Wasserstoffatom, eine niedere Alkylgruppe oder eine Phenylgruppe,
und deren Salze, sowie deren chirale Analoga
zur Herstellung einer pharmazeutischen Zusammensetzung, die zur Behandlung von Hautstörungen dient, die unter Rosacea, Pigmentstörungen, Barrierefunktionsstörungen und insbesondere Störungen der epidermalen Lipidsekretion, Wundheilungsstörungen, durch Corticosteroide oder Ulcera ausgelöste Hautatrophien; Störungen des Immunsystems; kardiovaskuläre Erkrankungen und/oder Störungen des Lipidstoffwechsels ausgewählt sind.

2. Kosmetische Verwendung einer ausreichenden Menge mindestens einer Verbindung der Formel (I): 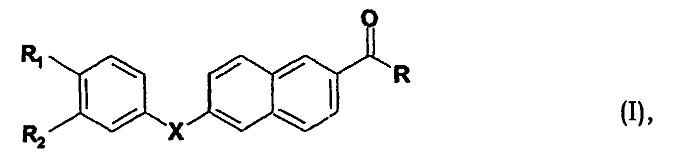 worin bedeuten:
R ein Wasserstoffatom oder eine Gruppe -OR₃,
wobei R₃ die unten angegebenen Bedeutungen hat,
X eine Gruppe C=O oder eine Gruppe C=N-OH,
R₁ und R₂, die gleich oder verschieden sind, ein Wasserstoffatom, eine geradkettige oder verzweigte Alkylgruppe mit 1 bis 12 Kohlenstoffatomen, eine Gruppe -OR₄, eine Gruppe -S(O)ₙR₄, eine Gruppe -OCOR₄ oder eine Gruppe -NHCOR₄,
wobei n und R₄ die unten angegebenen Bedeutungen haben,
wobei R₁ und R₂ zusammen mit dem benachbarten aromatischen Ring einen 5- oder 6-gliedrigen Ring bilden können, der gegebenenfalls mit Methylgruppen substituiert ist und/oder gegebenenfalls durch ein Sauerstoffatom, ein Schwefelatom, eine Sulfongruppe, oder eine Sulfoxidgruppe unterbrochen ist,
mit der Maßgabe, dass R1 und R2 mit dem benachbarten aromatischen Ring keinen 5-gliedrigen Ring bilden, der mit Methylgruppen substituiert ist, wenn X eine C=O-Gruppe bedeutet,
R₃ ein Wasserstoffatom, eine geradkettige oder verzweigte Alkylgruppe mit 1 bis 20 Kohlenstoffatomen oder eine Mono- oder Polyhydroxyalkylgruppe,
n 0, 1 oder 2,
R₄ ein Wasserstoffatom, eine niedere Alkylgruppe oder eine Phenylgruppe,
und deren Salze, sowie deren chirale Analoga
zur Vorbeugung und/oder Behandlung der intrinsischen (oder chronologischen) Alterung.

3. Verwendung nach Anspruch 1 oder 2, **dadurch gekennzeichnet, dass** die Verbindungen der Formel (I) als Aktivatoren der Rezeptoren vom Typ der PPARs verwendet werden.

4. Verwendung nach einem der Ansprüche 1 bis 3, **dadurch gekennzeichnet, dass** die Verbindungen der Formel (I) in Form von Alkali- oder Erdalkalisalzen, oder auch in Form von Zinksalzen oder als Salze eines organischen Amins vorliegen.

5. Verwendung nach einem der Ansprüche 1 bis 4, **dadurch gekennzeichnet, dass** die niederen Alkylgruppen unter Methyl, Ethyl, Isopropyl, Butyl, *t*-Butyl und Hexyl ausgewählt sind.

6. Verwendung nach einem der vorhergehenden Ansprüche, **dadurch gekennzeichnet, dass** die geradkettigen oder verzweigten Alkylgruppen mit 1 bis 20 Kohlenstoffatomen unter Methyl, Ethyl, Propyl, 2-Ethylhexyl, Octyl, Dodecyl, Hexadecyl und Octadecyl ausgewählt sind.

7. Verwendung nach einem der vorhergehenden Ansprüche, **dadurch gekennzeichnet, dass** die Monohydroxyalkylgruppen unter 2-Hydroxyethyl, 2-Hydroxypropyl oder 3-Hydroxypropyl ausgewählt sind.

8. Verwendung nach einem der vorhergehenden Ansprüche, **dadurch gekennzeichnet, dass** die Polyhydroxyalkylgruppen unter 2,3-Dihydroxypropyl, 2,3,4-Trihydroxybutyl, 2,3,4,5-Tetrahydroxypentyl oder Pentaerythrit ausgewählt sind.

9. Verwendung nach einem der Ansprüche 1 oder 2, **dadurch gekennzeichnet, dass** die Verbindungen ausgewählt sind unter: Verbindung 1: 6-(5,6,7,8-Tetrahydro-5,5,8,8-tetramethylnaphthalin-2-carbonyl)-naphthalin-2-carbonsäure
Verbindung 2: 6-(2,2-Dimethyl-chroman-6-carbonyl)-naphthalin-2-carbonsäure
Verbindung 3: 6-(4-*t*-Butyl-benzoyl)-naphthalin-2-carbonsäure Verbindung 4: 6-(5,6,7,8-Tetrahydro-naphthalin-2-carbonyl)-naphthalin-2-carbonsäure
Verbindung 5: 6-(4,4-Dimethyl-chroman-6-carbonyl)-naphthalin-2-carbonsäure
Verbindung 6: 6-[Hydroxyimino-(5,5,8,8-tetramethyl-5,6,7,8-tetrahydro-naphthalin-2-yl)-methyl]-naphthalin-2-carbonsäure.

10. Verwendung nach einem der Ansprüche 1 oder 2, **dadurch gekennzeichnet, dass** die der Formel (I) entsprechende Verbindung die 6-(4-*t*-Butyl-benzoyl)-naphthalin-2-carbonsäure ist.

11. Verwendung nach einem der Ansprüche 1 oder 3, **dadurch gekennzeichnet, dass** es sich bei den Pigmentstörungen um Hyperpigmentierung, Melasma, Hypopigmentierung oder Vitiligo handelt.

12. Verwendung nach Anspruch 11, **dadurch gekennzeichnet, dass** die Pigmentstörungen nicht in Verbindung zu den schädlichen Wirkungen der Sonne stehen.

13. Verwendung nach einem der Ansprüche 1 oder 3, **dadurch gekennzeichnet, dass** es sich bei den seborrhoischen Funktionsstörungen um Hyperseborrhoe oder seborrhoische Dermatitis handelt.

14. Verwendung nach einem der Ansprüche 1 oder 3, **dadurch gekennzeichnet, dass** die Barrierefunktionsstörungen und genauer die Störungen der epidermalen Lipidsekretion Störungen der Haut von frühgeborenen Kindern, die vor der 33. Woche geboren wurden, Lippenfissuren oder Blasen aufgrund von mechanischer Reibung sind.

15. Verwendung nach einem der Ansprüche 1 oder 3, **dadurch gekennzeichnet, dass** die Wundheilungsstörungen keloide oder hypertrophe Narben sind.

16. Verwendung nach einem der Ansprüche 1 oder 3, **dadurch gekennzeichnet, dass** die Ulcera durch chemische oder thermische Verbrennungen bedingte Ulcera und Erosionen, bullöse Störungen, vaskuläre oder ischämische Störungen inklusive venöser, artherieller, embolischer oder diabetischer Ulcera sind.

17. Verwendung nach einem der Ansprüche 1 oder 3, **dadurch gekennzeichnet, dass** die Störungen des Immunsystems Autoimmunerkrankungen wie Diabetes vom Typ 1, multiple Sklerose, Lupus und Erkrankungen vom Lupustyp, Glomerulonephritis, oder selektive Dysfunktionen des Immunsystems wie Aids sind.

18. Verwendung nach einem der Ansprüche 1 oder 3, **dadurch gekennzeichnet, dass** es sich bei den Erkrankungen des kardiovaskulären Systems um Arteriosklerose oder Bluthochdruck handelt.

19. Verwendung nach einem der Ansprüche 1 oder 3, **dadurch gekennzeichnet, dass** die Lipidstoffwechselstörungen Adipositas, Hyperlipidämie, oder nicht insulinabhängiger Diabetes sind.

20. Verwendung nach einem der vorhergehenden Ansprüche, **dadurch gekennzeichnet, dass** die Verbindungen der Formel (I) mit anderen Verbindungen mit einer Aktivität vom Retinoidtyp, mit D-Vitaminen und deren Derivaten, mit Corticosteroiden, mit Radikalfängern für freie Radikale, mit α-Hydroxy- oder α-Ketosäuren und deren Derivaten, oder mit Ionenkanalblockern kombiniert werden.

21. Verwendung nach einem der vorhergehenden Ansprüche, **dadurch gekennzeichnet, dass** die Zusammensetzung enteral oder parenteral verabreicht wird.

22. Verwendung nach einem der Ansprüche 1 bis 17, **dadurch gekennzeichnet, dass** die Zusammensetzung topisch oder okular verabreicht wird.

23. Verwendung nach dem vorhergehenden Anspruch, **dadurch gekennzeichnet, dass** die Verbindungen der Formel (I) in einer Konzentration von 0,001 bis 10 Gew.-%, bezogen auf das Gesamtgewicht der Zusammensetzung, verwendet werden.

24. Verfahren zur kosmetischen Behandlung, um die Barrierefunktion wiederherzustellen und insbesondere um den Hautlipidstoffwechsel zu regulieren und/oder wiederherzustellen, **dadurch gekennzeichnet, dass** eine Zusammensetzung, die mindestens eine Verbindung der Formel (I) in einer wirksamen Menge enthält, auf die Haut aufgetragen wird.

25. Verfahren zur kosmetischen Behandlung, um die Barrierefunktion wiederherzustellen und insbesondere um den Hautlipidstoffwechsel zu regulieren und/oder wiederherzustellen, **dadurch gekennzeichnet, dass** eine Zusammensetzung, die mindestens eine Verbindung der Formel (I) als Aktivator für Rezeptoren vom Typ der PPARs in einer wirksamen Menge enthält, auf die Haut aufgetragen wird.

26. Verfahren zur kosmetischen Behandlung nach einem der Ansprüche 24 oder 25, **dadurch gekennzeichnet, dass** die Konzentration der Verbindungen der Formel (I) im Bereich von 0,001 bis 3 Gew.-%, bezogen auf die gesamte Zusammensetzung, liegt.
